(19) Europäisches Patentamt
European Patent Office
Office européen des brevets

(11) Numéro de publication: **0 090 736**
**A1**

(12) **DEMANDE DE BREVET EUROPEEN**

(21) Numéro de dépôt: **83400629.8**

(51) Int. Cl.³: **C 07 J 41/00**, A 61 K 31/565

(22) Date de dépôt: **25.03.83**

(30) Priorité: **29.03.82 FR 8205297**

(43) Date de publication de la demande: **05.10.83**
**Bulletin 83/40**

(84) Etats contractants désignés: **AT BE CH DE FR GB IT LI LU NL SE**

(71) Demandeur: **Etablissement Public dit: CENTRE *NATIONAL DE LA RECHERCHE SCIENTIFIQUE* (CNRS), 15, Quai Anatole France, F-75007 Paris (FR)**

(72) Inventeur: **Imbach, Jean Louis, 1108 rue des Sorbes, F-34000 Montpellier (FR)**
Inventeur: **Chavis, Claude**, 38 rue des Pins, F-34980 Saint **Gely Du Fesc (FR)**

(74) Mandataire: **Warcoin, Jacques et al, Cabinet Régimbeau 26, avenue Kléber, F-75116 Paris (FR)**

(54) **Nouvelle nitrosourée stéroide à propriété oncostatique, procédé pour sa préparation et son utilisation à titre de médicament.**

(57) La présente invention concerne le 17β-(N-2-chloroéthyl-N'-nitrosouréyl)-5-androsten-3β-ol.

Ce composé est utile comme médicament antitumoral.

EP 0 090 736 A1

ACTORUM AG

# NOUVELLE NITROSOUREE STEROIDE A PROPRIETE ONCOSTATIQUE, PROCEDE POUR SA PREPARATION ET SON UTILISATION A TITRE DE MEDICAMENT.

La présente invention concerne une nouvelle nitrosourée stéroïde à propriété oncostatique, un procédé pour sa préparation et l'utilisation de cette nitrosourée à titre de médicament.

Parmi les substances antitumorales connues actuellement, les agents alkylants et les hormones stéroïdes constituent deux classes de drogue de grande importance dont les activités cytotoxiques sont largement exploitées.

Il était donc logique de tester un nouveau type de composés anticancéreux actifs contre des tumeurs hormono-dépendantes mettant à profit une possible combinaison dans un même produit d'une partie alkylante cytotoxique et d'un support lipophilique ayant une activité hormonale qui présenterait ainsi une grande sélectivité à l'égard du récepteur correspondant. On pense que dans une telle association l'agent alkylant peut être amené sur un tissu cible particulier ou peut désactiver le récepteur stéroïdien.

Le composé selon la présente invention est un produit de ce type mais il présente la caractéristique tout à fait exceptionnelle d'avoir la meilleure activité oncostatique sur la leucémie $L_{1210}$ parmi tous les produits testés jusqu'à présent.

Le composé selon la présente invention est le 17β-(N-2-chloroéthyl-N'-nitrosouréyl)-5-androsten-3β-ol de formule :

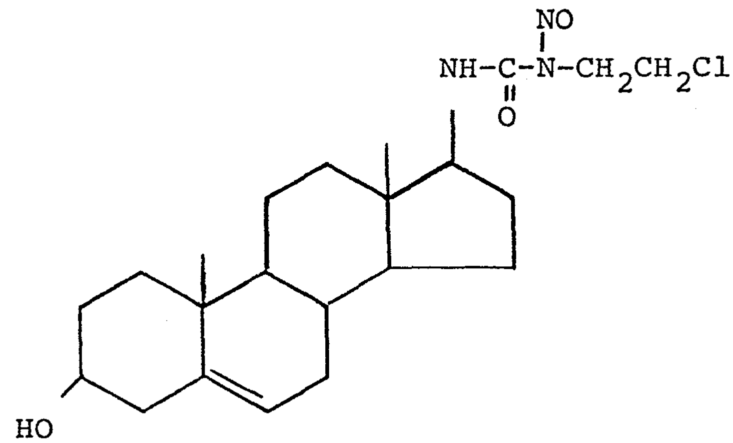

ainsi que les esters et éthers correspondants en position 3 et le N-2-chloroéthyl-N-nitrosocarbamate en position 3 correspondant.

Le composé selon la présente invention peut être préparé par réaction du 17β-amino-5-androsten-3β-ol sous forme libre, activé et/ou protégé, avec un N-2-chloroéthyl-N-nitrosocarbamate activé, en particulier le carbamate de p-nitrophényle, ou bien un autre nitrosocarbamate activé décrit notamment dans le brevet français n° 80 16453 du 25 juillet 1980, selon la réaction suivante :

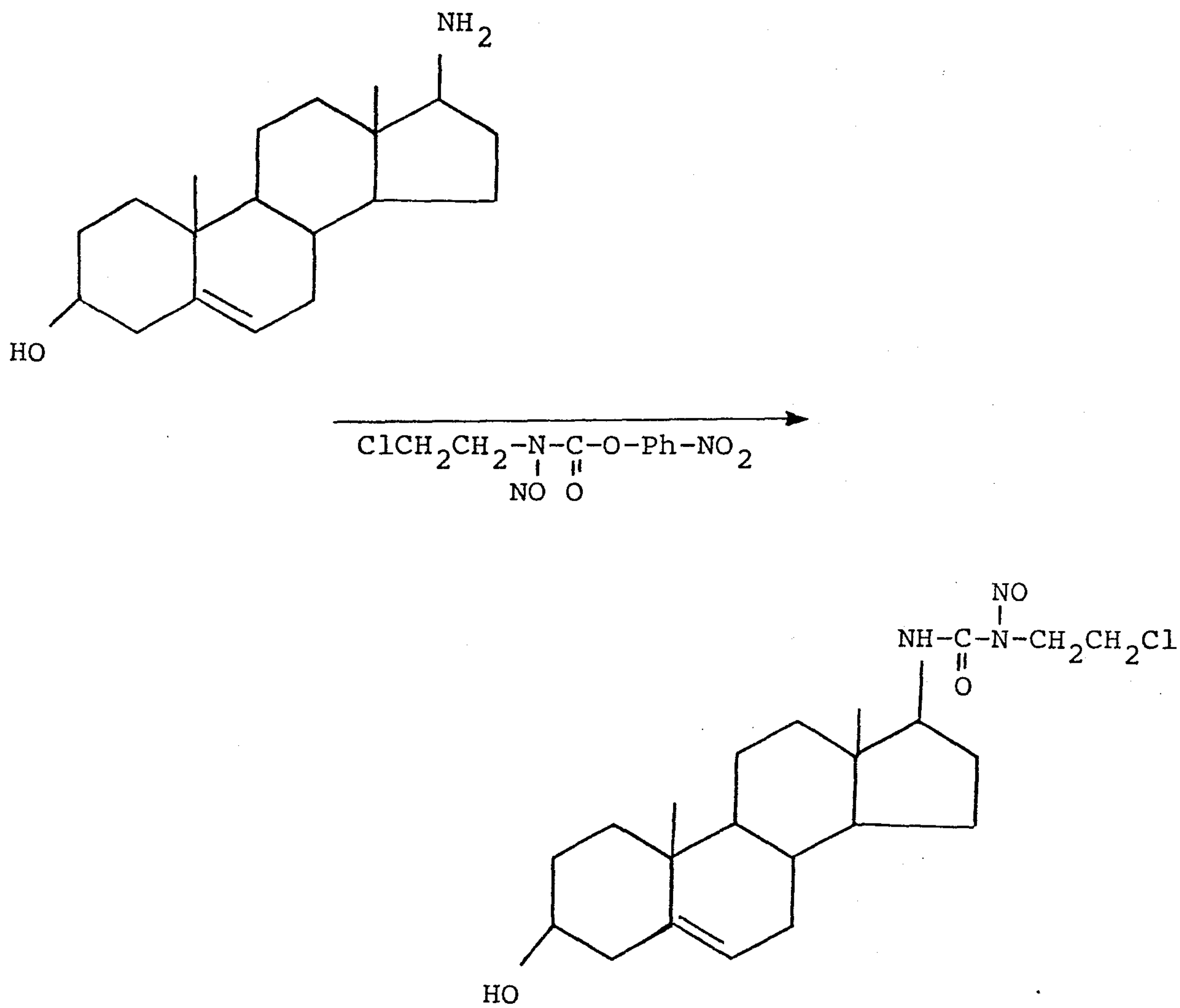


L'androsténol de départ est un produit connu et décrit dans G.R. PETTIT, R.L. SMITH, A.K. DAS GUPTA et J.L. OCCOLOWITHZ, Can. J. Chem. 1967n 45, 501-507.

Les éthers et/ou esters correspondants peuvent être préparés par des procédés connus et ils correspondent plus particulièrement à des esters ou éthers comportant des chaînes alkyles, aralkyles ou aryles substituées ou non, en particulier par des radicaux lipophiles ou par des radicaux hydrophiles tels que des radicaux hydroxy ou amino.

Parmi les éthers ou esters, il faut citer également ceux comportant la structure d'un ose ou des structures apparentées.

Ces éthers permettent de modifier la balance hydrophile-lipophile du composé et ainsi de modifier son activité

Le composé selon la présente invention est utile comme médicament présentant une activité oncostatique.

Le composé selon la présente invention est donc utilisable pour le traitement des tumeurs liquides ou solides, en particulier les leucémies.

Il est évident pour l'homme de métier que les doses thérapeutiques à appliquer dépendent considérablement de la nature et de l'état d'évolution de la tumeur à traiter et de l'état général du patient. Toutefois, dans le cas particulier du composé selon l'invention, l'index thérapeutique étant très élevé, les dosages utilisables peuvent donc varier dans de larges limites.

Ainsi, les doses journalières pourront varier par exemple de 50 à 200 mg/kg/jour sans que cela soit limitatif.

La présente invention sera mieux comprise à la lecture des exemples ci-après qui illustrent certains aspects particuliers de l'invention.

EXEMPLE 1

17β-(N-2-chloroéthyl-N'-nitrosouréyl)-5-androsten-3β-ol

A 0,21 g (0,715 mmole) de 17β-amino-5-androsten-3β-ol dans 5 ml de pyridine anhydre à -10°C est ajouté 0,226 g (0,828 mmole) de N-2-chloroéthyl-N-nitrosocarbamate de p-nitrophényle. L'agitation est maintenue pendant 3 heures à cette température puis le mélange réactionnel est évaporé sous vide puis coévaporé trois fois avec 20 ml du mélange toluène-alcool (1/1).

L'huile résiduelle est cristallisée dans l'éthanol (5 ml) pour donner le composé du titre sous forme de cristaux jaunes (0,29 g, 93 %).
F = 139-141°C ; c.c.m. : $R_f$ = 0,36 (chloroforme-éthanol 9,5/0,5) ; $(\alpha)_D^{20}$ - 40 (cl, $CHCl_3$).
IR ($CHCl_3$), 3600, 3420, 2940-2860, 1720 (C=O), 1460 (N-NO), 1345, 1330, 1300, 1165, 1085, 970, 900, $^1H$ NMR ($CDCl_3$) : 0,74 (s, 3H, 18-H), 1,00 (s, 3H, 19-H), 1,62 (s, OH), 3,47 (t, 2H, $CH_2$-N-NO, J = 6,5 Hz), 4,16 (t, 2H, $CH_2$-Cl), 3,96 (m, 1H, 17α-H), 5,34 (m, 1H, 6-H), 6,79 (d, 1H, N-H, J = 9Hz). Masse : m/e/I, 316/27 $(M-ClCH_2CH_2N = NOH)^+$, 299/31 $M-(H_2O + ClCH_2CH_2-N-NO^+)$, 298/100 $(M-ClCH_2CH_2-N = NOH)^{+\cdot} - H_2O$, 283/84 $(298-CH_3)^{+\cdot}$ Anal calc. pour $C_{22}H_{34}N_3O_3Cl$ : C 62,32 ; H 8,08 ; N 9,91 ; trouvé C 62,11 ; H 8,10 ; N 9,77.

Le point de fusion est pris en capillaire et n'est pas corrigé. Les chromatographies sur couche mince ont été réalisées sur feuilles d'aluminium recouvertes de gel de silice 60F254 Merck. Le spectre IR a été enregistré sur un spectrographe Perkin Elmer 450. Le pouvoir rotatoire a été pris sur un appareil Roussel-Jouan. Le spectre de masse a été obtenu avec un instrument Jeol JMS D100. Le spectre de RMN du $^1H$ a été enregistré sur un spectrographe Varian HA 100 avec le TMS comme référence interne. (d = doublet, t = triplet, m = multiplet).

EXEMPLE 2

Etude de l'activité du composé selon la présente invention sur la leucémie $L_{1210}$

Le composé selon la présente invention a été étudié sur la leucémie $L_{1210}$ en comparaison avec d'autres nitrosourées présentant également des propriétés oncostatiques. Il s'agit essentiellement de la BCNU, la CCNU, la Me CCNU, la RFCNU, la RPCNU et la Chlorozotocine.

Ces composés, ainsi que le XPCNU, sont bien connus et décrits par exemple dans Biomedicine Express, vol. 23, n° 10 du 10 décembre 1975, pages 410-413.

Leucémie $L_{1210}$

Pour chaque composé, $10^5$ cellules leucémiques ont été inoculées à 80 souris ($DBA_2$ - $C_{57}BL$ 100), par voie intrapéritonéale.

Les traitements ont débuté un jour après la transplantation et ont été poursuivis les cinquième et neuvième jours. Les dérivés devant être testés sont administrés en suspension dans l'huile d'olive à 70 souris. Les doses injectées sont variables, chaque souris étant pesée individuellement. Les 10 souris restantes constituent le groupe témoin et reçoivent, en placébo, des volumes équivalents d'huile d'olive.

Le premier jour, les nitrosourées testées sont administrées, en doses variables, aux souris traitées.

Les cinquième et neuvième jours, les injections sont renouvelées sans signe macroscopique de toxicité.

Pour chaque composé, les activités oncostatiques ont été déterminées pour une dose spécifique et varient en fonction de la quantité administrée. Lorsque l'activité oncostatique est supérieure à 125 et si la différence des groupes témoins et traités à une signification statistique (test de WILCOXON), le composé est considéré comme étant actif à la dose utilisée.

Les résultats obtenus sont rassemblés dans la figure ci-annexée.

La figure ci-annexée indique en fonction des doses de principe actif appliquées en milligramme par kilogramme le rapport T/C défini comme suit :

T/C = moyenne de survie des groupes traités / moyenne de survie des groupes témoins x 100

La mention ∞ indique que plus de 50 % des animaux traités sont guéris.

Il ressort de l'étude de la figure ci-annexée que le produit selon l'invention présente très nettement la meilleure activité oncostatique sur la leucémie $L_{1210}$ parmi tous les produits testés jusqu'à présent.

On a, en outre, indiqué ci-après dans le tableau pour les composés connus et pour le composé selon la présente invention les MEDI, c'est-à-dire les doses médianes, les doses optimums, les $DL_{50}$ et par là l'index thérapeutique de ces composés.

NITROSOUREES AYANT UN MEDI SUR LA $L_{1210}$

| Nom | MEDI (mg/kg) | Dose optimale (mg/kg) | $DL_{50}$ (mg/kg) | Index thérapeutique |
|---|---|---|---|---|
| RFCNU | 15-30 | 20 | 90 | 4,5 |
| RPCNU | 10-15 | 15 | 50 | 3,3 |
| XPCNU | 10-15 | 15 | | |
| CZT | 10-20 | 15 | 35 | 2,33 |
| Composé ex. 1 | 50-200 | 125 | ~700 | 5,6 |

On constate qu'à l'activité déterminée précédemment s'ajoute la dose médiane la plus grande jamais observée et une $DL_{50}$ aiguë de 700 mg/kg qui est la plus avantageuse parmi toutes les autres nitrosourées, ce qui conduit à l'indice thérapeutique le plus élevé jamais obtenu jusqu'à présent pour ce type de composé oncostatique.

## REVENDICATIONS

1) le 17β-(N-2-chloroéthyl-N'-nitrosouréyl)-5-androsten-3β-ol, les éthers ou esters en position 3 correspondants ainsi que le N-2-chloroéthyl-N-nitrosocarbamate en position 3.

2) Procédé de préparation du composé selon la revendication 1, caractérisé en ce qu'on fait réagir le 17β -amino-5-androsten-3β-ol sous forme libre,activé et/ou protégé avec un N-2-chloroéthyl-N-nitrosocarbamate activé.

3) Procédé selon la revendication 2, caractérisé en ce qu'on fait réagir le 17β-amino-5-androsten-3β-ol avec le N-2-chloroéthyl-N-nitrosocarbamate de p-nitrophényle.

4) A titre de médicament nouveau, un composé selon la revendication 1.

5) Composition pharmaceutique, caractérisée en ce qu'elle comporte à titre de principe actif au moins un composé selon la revendication 1.

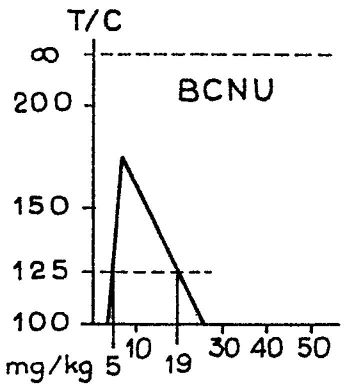
T/C
∞
200
150
125
100
BCNU
mg/kg 5 10 19 30 40 50

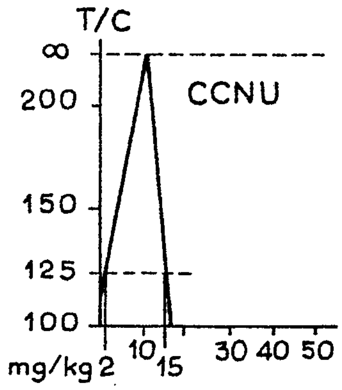
T/C
∞
200
150
125
100
CCNU
mg/kg 2 10 15 30 40 50

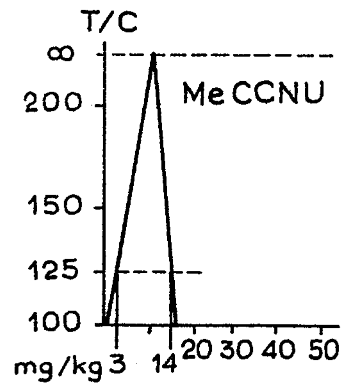
T/C
∞
200
150
125
100
Me CCNU
mg/kg 3 14 20 30 40 50

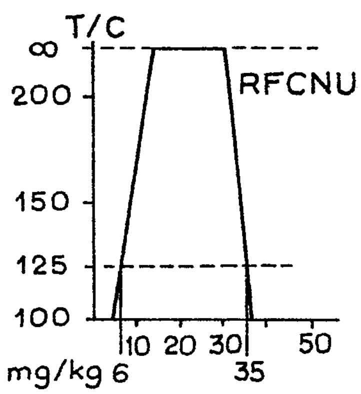
T/C
∞
200
150
125
100
RFCNU
mg/kg 6 10 20 30 35 50

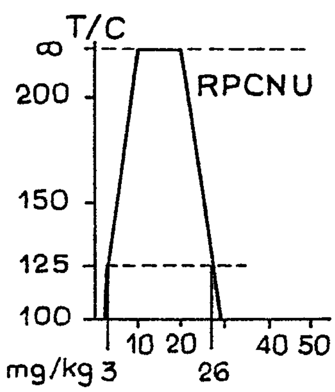
T/C
∞
200
150
125
100
RPCNU
mg/kg 3 10 20 26 40 50

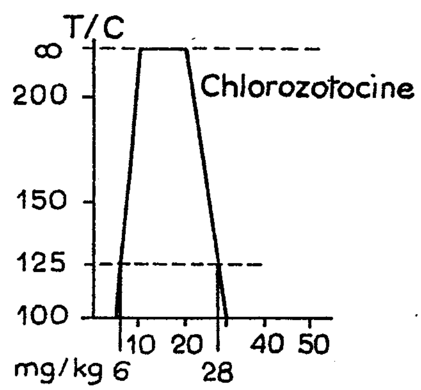
T/C
∞
200
150
125
100
Chlorozotocine
mg/kg 6 10 20 28 40 50

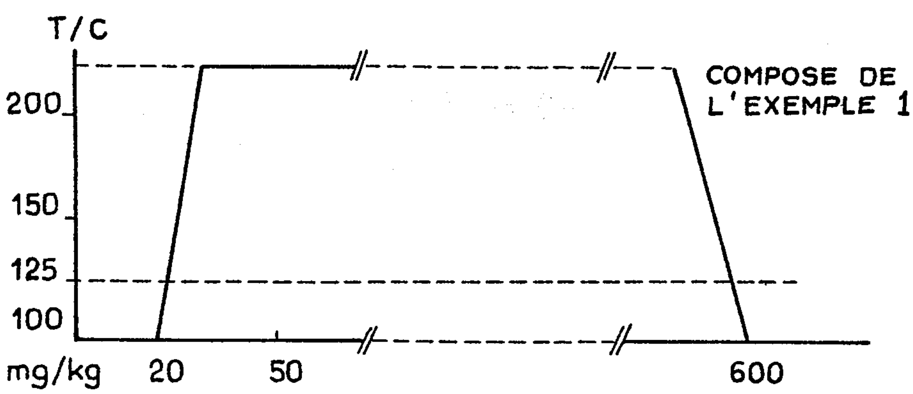
T/C
200
150
125
100
COMPOSE DE
L'EXEMPLE 1
mg/kg 20 50 600

Office européen des brevets

# RAPPORT DE RECHERCHE EUROPEENNE

Numéro de la demande

EP 83 40 0629

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X,Y | STEROIDS, vol. 39, no. 2, février 1982, pages 129-147, Holden Day, San Francisco, USA C. CHAVIS et al.: "New steroidal nitrosoureas" * Page 131, composé 5; page 137; pages 142-147 * | 1-5 | C 07 J 41/00<br>A 61 K 31/565 |
| | --- | | |
| Y | JOURNAL OF MEDICINAL CHEMISTRY, vol. 22, no. 2, février 1979, pages 200-202, American Chemical Society, Washington, USA H.Y.P. LAM et al.: "Synthesis of steroidal nitrosoureas with anti-tumor activity" * En entier * | 1,4,5 | |
| | ----- | | **DOMAINES TECHNIQUES RECHERCHES (Int. Cl. 3)** |
| | | | C 07 J 41/00 |

Le présent rapport de recherche a été établi pour toutes les revendications

| Lieu de la recherche | Date d'achèvement de la recherche | Examinateur |
|---|---|---|
| LA HAYE | 15-06-1983 | HENRY J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant